Europäisches Patentamt

(19) European Patent Office

Office européen des brevets

(11) Veröffentlichungsnummer: **0 001 124**

**A1**

(12) **EUROPÄISCHE PATENTANMELDUNG**

(21) Anmeldenummer: **78100865.1**

(22) Anmeldetag: **11.09.78**

(51) Int. Cl.²: **A 61 K 31/20**
**A 61 K 31/64, A 61 K 37/02**
**A 61 K 37/26, A 61 K 9/00**
**A 61 K 9/06, A 61 K 9/20**

(30) Priorität: **12.09.77 DE 2740953**

(43) Veröffentlichungstag der Anmeldung:
**21.03.79 Patentblatt 79/6**

(84) Benannte Vertragsstaaten:
**CH FR GB NL SE**

(71) Anmelder: THERA Gesellschaft für Patentverwertung mbH
Schmidschneiderstrasse 15
D-8036 Herrsching(DE)

(72) Erfinder:
Die Erfinder haben auf ihre Nennung verzichtet

(74) Vertreter: Wuesthoff, Franz, Dr.-Ing. et al,
Patentanwälte Wuesthoff -v. Pechmann-Behrens-Goetz
Schweigerstrasse 2
D-8000 München 90(DE)

(54) **Neue Arzneimittelmischungen zur Verbesserung der Glucoseverwertung in den Zellen und Senkung des Blutzuckerspiegels.**

(57) Neue Stoffgemische aus Prostaglandin $E_1$ und/oder $E_2$ mit anderen Wirkstoffen ergeben Arzneimittel, die eine Verbesserung der Glucoseverwertung der Zellen und eine Verminderung des Blutzuckerspiegels im Organismus bewirken. In Gemischen mit Glucose, Fructose, Xylit und/oder Aminosäuren oder auch zusammen mit eiweißfreien Extraktstoffen des Blutes junger Kälber ergeben diese Prostaglandine neuartige Infusionslösungen. In Gelees oder Salben verarbeitet, eignet sich die Kombination mit den Blutextraktstoffen auch zur Anwendung als lokales Wundheilmittel. Die Kombination mit oralen Antidiabetica oder mit Insulin ergibe verbesserte blutzucker-senkende Arzneimittel.

EP 0 001 124 A1

# 0001124

Neue Arzneimittelmischungen zur Verbesserung der Glucoseverwertung in den Zellen und Senkung des Blutzuckerspiegels

Die vorliegende Erfindung betrifft neue Stoffgemische, die als Arzneimittel Verwendung finden, da sie aufgrund eines Gehalts an bestimmten Prostaglandinen den Blutzuckerspiegel erniedrigen bzw. die Verwertung der Glucose in den Zellen fördern.

Bekanntlich ist bei den Patienten mit Diabetes mellitus der Blutzuckerspiegel stark erhöht. Durch regelmäßige Injektionen von dem aus tierischem Pankreas gewonnenen Insulin kann eine Normalisierung und Kontrolle des Glucosegehalts im Blut erreicht werden. Auch orale Antidiabetica, insbesondere die der Benzolsulfonylharnstoffreihe, können in vielen Fällen mit Erfolg bei der Behandlung der Diabetes mellitus verwendet werden. Allerdings haben beide Mittel zur Bekämpfung dieser Krankheit ihre Nachteile. Die Produktion des Insulins ist begrenzt und seine Applikation führt häufig zu einer Hypoglykämie. Auch werden Allergien und Resistenzerscheinungen berichtet (Mehnert & Schöffling, Diabetologie in Klinik und Praxis, Thieme-Verlag, Stuttgart 1974, S. 283 - 298). Bei den oralen Antidiabetica müssen bei vielen Patienten immer höhere Dosierungen angewandt werden, um die gewünschte Wirkung im Organismus zu erreichen, wobei aber bei grösseren Gaben gastro-intestinale Störungen auftreten können und auch die gewünschten Effekte der Blutzuckersenkung nicht mehr erreicht werden. Bei Patienten mit erhöhten Blutzuckerwerten treten auch häufig Entzündungen im Hautbereich auf, was auf eine gestörte Glucose-

- 2 -

0001124

verwertung in den betreffenden Zellen zurückgeführt werden kann. Daher besteht ein Bedürfnis nach neuen verbesserten Arzneimitteln, die die Glucoseverwertung in den Zellen verbessern bzw. den Blutzuckerspiegel erniedrigen.

Es wurde nun überraschenderweise festgestellt, daß neue Stoffgemische, deren eine Komponente aus Prostaglandin $E_1$ und/oder $E_2$ besteht, bei lokaler, oraler oder perkutaner Applikation eine Steigerung der Glucoseverwertung der Zellen und Erniedrigung des Blutzuckerspiegels bereits bei relativ geringer Dosierung bewirken.

Die Prostaglandine stellen $C_{20}$-ungesättigte Carbonsäuren dar, die besonders durch einen zwischen zwei Seitenketten eingeschobenen Fünfring charakterisiert sind. Bisher sind mehrere verschiedene Prostaglandine bekannt, deren wichtigste als Prostaglandine $A_1$, $A_2$, $E_1$, $E_2$ und F bezeichnet werden und die sich in ihren physiologischen Wirkungen unterscheiden. In "Umschau", 1975, S. 197 - 202, sind einige therapeutische Anwendungsmöglichkeiten erwähnt und in Acta physiol. scand., 1966, S. 141 - 151 und 185 - 193, ist der Einfluß von Prostaglandin $E_1$-Gaben auf die freien Fettsäuren des Plasmas und den Blutzuckerspiegel beschrieben. Ein klares Bild haben diese Untersuchungen nicht ergeben. Bei manchen Versuchen zeigte sich ein Ansteigen des Blutzuckers, in anderen nicht (vgl. auch Clin. Research, 1967, 15, S. 410, wo über ein Ansteigen des Blutzuckerspiegels referiert ist).

Die wesentlichen physiologischen Funktionen der Prostaglandine sind bisher noch nicht voll geklärt. Im Hinblick auf die mit ihnen erreichbaren Effekte wurden für einzelne Prostaglandine bisher in der klinischen Therapie folgende Nutzungsanwendungen gezogen: Stimulation der Kontraktionstätigkeit des Uterus zur Förderung der Wehen des schwangeren Uterus; Relaxation der glatten Muskulatur in Bronchien und Gefäßen,

0001124

d.h. a) als Broncholytikum bei Asthma bronchiale oder b) als antipressorische Substanz bei Hypertonie; Schutzfunktion auf die Magenschleimhaut bei der Therapie des Ulcus ventriculi et duodeni.

Die durch die Prostaglandine erreichten Stoffwechselwirkungen wurden bisher noch nicht in der Klinik therapeutisch eingesetzt. Aus zahlreichen biochemischen Untersuchungen ist eine Hemmung der Lipolyse, wohl über das cAMP-System, bekannt. Damit in Verbindung steht wohl auch der Effekt auf den Glucosestoffwechsel einiger Prostaglandine in in vitro-Systemen, d.h. bei Zellkulturen und isoliertem Fettgewebe, da die Zelle wegen Hemmung des Fettstoffwechsels nun vermehrt auf Kohlenhydratverwertung angewiesen ist. Dieser Effekt ist nach bisheriger Anschauung für den Gesamtorganismus ohne Relevanz, da hier am Menschen und an Hunden nach intravenöser Gabe von Prostaglandin sogar ein Anstieg des Blutzuckerspiegels gefunden wurde, der über eine Stimulation der Freisetzung von Katecholaminen erklärt wird.

Es muß nun als sehr überraschend bezeichnet werden, daß im Gegensatz zu diesen Befunden festgestellt wurde, daß die beiden Prosta-glandine $E_1$ und $E_2$ in bestimmten Stoffwechselzuständen eine potente klinisch relevante Verringerung des Glucoseblutspiegels des Organismus bewirken, eine Wirkung, die bei den anderen Prostaglandinen, wie z.B. dem Prostaglandin F, nicht auftritt. Nähere Untersuchungen dieses Effektes haben gezeigt, daß er auf einer direkten Wirkung der Prosta-glandine $E_1$ und $E_2$ auf den Glucosetransport über die Muskelzellmembranen beruht, wie man dies bisher nur für das Hormon Insulin kannte.

Dieser den Blutzuckerspiegel im Blut senkende Effekt ist auch deshalb überraschend, da Insulin sowie die oral zu applizierenden Antidiabetica vom Benzolsulfonylharnstofftyp keine der bisher klinisch bekannten Effekte der Prostaglandine (s.o. Uteruskontraktion, Broncholyse, Anti-

0001124

hypertensiv, Protektion der Magenschleimhaut) besitzen. Es war daher nicht zu erwarten, daß Arzneimittelgemische, die als Wirkstoffkomponente eine Substanz mit derartigen physiologischen Wirkungen enthalten, einen Einfluß auf den Transport von Glucose durch die Zellmembran haben und auf diese Weise den Blutzuckerspiegel bei Diabetespatienten senken könnten.

Für die praktische Anwendung der neuartigen Wirkung der Prostaglandine bei der Senkung des Blutzuckerspiegels eignet sich besonders die Zugabe zu glucosehaltigen oder andere Substanzen enthaltenden Infusionslösungen. Diese Kombinationslösungen können im allgemeinen je Liter Infusionslösung 100 bis 300 g Glucose und/oder 50 bis 100 g Fruktose oder 50 bis 100 g Xylit sowie erfindungsgemäß 0,3 bis 3,0 mg, vorzugsweise etwa 1,0 mg Prostaglandin $E_1$ oder 1,0 bis 20 mg, vorzugsweise etwa 10 mg Prostaglandin $E_2$, enthalten. Für parenterale Ernährungszwecke können die Lösungen auch Aminosäuren bzw. andere Zucker, Alkohole, Mineralsalze und andere übliche Zusätze enthalten. Als besonders geeignet hat sich eine Infusionslösung gezeigt, die je Liter die angegebenen Mengen an Prostaglandin $E_1$ oder $E_2$ enthält, sowie folgende in der Tabelle I angegebene Aminosäuren.

## TABELLE I

0001124

Bereiche für die Aminosäurenlösung:  g/l

| | |
|---|---|
| Isoleucin | 1 - 6 |
| Leucin | 2 - 10 |
| Lysin | 2 - 10 |
| Methionin | 2 - 5 |
| Phenylalanin | 2 - 6 |
| Threonin | 1 - 5 |
| Tryptophan | 0.5 - 2 |
| Valin | 1 - 6 |
| Arginin | 2 - 10 |
| Histidin | 1 - 10 |
| Asparaginsäure | 0.5 - 4 |
| Glutaminsäure | 4 - 20 |
| Glycin | 2 - 20 |
| Alanin | 5 - 15 |
| Tyrosin | 0.1 - 1 |
| Prolin | 5 - 15 |
| Serin | 1 - 4 |
| Cystin | 0.1 - 1 |
| Ornithin | 1 - 4 |
| Asparagin | 1 - 4 |

0001124

Für die praktische Anwendung ist das neue Stoffgemisch in Form der Kombination von Glucose und Prostaglandinen bei den Infusionslösungen von besonderer Bedeutung, da hierbei der Blutzuckerspiegel niedrig gehalten wird durch Förderung des Glucosetransports aus dem Blutstrom in die Muskelzelle, so daß trotz erhöhter Glucosezufuhr, insbesondere bei hohen Infusionsgeschwindigkeiten die Konzentrationswerte im Blut nicht in unerwünschter Weise ansteigen.

Die Prostaglandine $E_1$ und $E_2$ können auch mit bestimmten Extraktstoffen aus dem Bluteiweiß junger Kälber erfindungsgemäße Arzneimittelgemische bilden, um eine Verbesserung der mit diesen Wirkstoffen erzielten Heilungen zu erreichen. Diese Extraktstoffe, die bei Durchblutungs- und Stoffwechselstörungen Anwendung finden können, vgl. Med. Welt, Bd. 19, S. 198 (1968), sind u.a. unter der geschützten Bezeichnung "Actihaemyl" der Firma Solko und unter dem Namen "Actovegin" der Firma Hormonchemie im Handel. Sie können oral, intravenös oder intraarteriell infundiert oder intramuskular injiziert oder als Salbe appliziert werden. Diese potenzierende Wirkung der Prostaglandine kann auch bei dieser neuen Kombination auf die Förderung des Stoffwechsels durch verstärkten Transport von Glucose in den mangelhaft versorgten Geweben zurückgeführt werden, wodurch die Heilwirkung der Blutextrakte unterstützt und wesentlich verstärkt wird. Auch dies war aufgrund der bisherigen Kenntnisse über die Prostaglandin—Wirkung nicht zu erwarten.

In der nachfolgenden Tabelle II sind die anzuwendenden Konzentrationen der erfindungsgemäßen Arzneimittelmischungen angegeben.

0001124

## TABELLE II

| | Blut-Extrakt | Prostaglandin $E_1$ | $E_2$ |
|---|---|---|---|
| Gelee (1) | 200 - 1000 mg;(400mg) | 0,45-4,5mg; (1,5 mg) | 0,1-30mg (15mg) |
| Salbe (1) | 50 - 250 mg;(100 mg) | 0,1-1,5 mg; (0,5 mg) | 0,05-10mg ( 5mg) |
| Intravenöse Infusions- lösung (2) | 0,3-2,5gr; (0,5gr) | 0,02-0,25 mg (0,08 mg) | 0,008-1,7 mg (0,8mg) |
| Intraarterielle Infusions- lösung (3) | 50-500 mg; (100mg) | 40-200 ng; (100 ng) | 0,05-2,3 $\mu$g (1 $\mu$g) |

(1) ad 100 ml eines üblichen Basismaterials für die lokale Applikation

(2) in 250 ml Aqua dest.

(3) in 100 ml Aqua dest.

Die bevorzugten Dosierungen sind in Klammern angegeben.

- 8 -

0001124

Auch die Kombination der Prostaglandine mit oralen Antidiabetika ergibt erfindungsgemäße Arzneimittelgemische. Insbesondere mit Substanzen der Benzolsulfonylharnstoffreihe wird eine Verminderung des Blutzuckerspiegels erreicht, die nicht durch eine bloße Addition der Wirkungen dieser Mittel erklärt werden kann, da die Effekte größer sind als die Additionswerte der Blutzuckersenkung der einzelnen Komponenten. Hierbei haben sich für Granulatpräparate bzw. Tablettiermassen Dosierungen von 100 mg bis 10 g für Prostaglandin $E_2$ und 30 mg bis 2 g für das Prostaglandin $E_1$ je 100 g Tolbutamid geeignet erwiesen. Eine Tablette dieses erfindungsgemäßen Arzneimittelgemisches soll etwa 0,35 g Tolbutamidsubstanz oder einen anderen oralen Antidiabeteswirkstoff in äquivalenter Dosierung enthalten. Für die Behandlung werden etwa 3 bis 4 Tabletten je Tag appliziert.

Auch die Kombination mit Insulin, sowohl mit Altinsulin als auch mit Depotinsulin, ergibt erfindungsgemäße Stoffmischungen, mit denen sich der erkannte Blutzucker-senkende Effekt der Prostaglandine vorteilhaft für die Diabetesbehandlung auswerten läßt, wobei in 1 ml Injektionslösung mit 40 E Insulin erfindungsgemäß 1,0 bis 8 mg Prostaglandin $E_2$ bzw. 0,1 bis 1,2 mg Prostaglandin $E_1$ enthalten sein sollen. Diese neue Arzneimittelkombination ermöglicht eine Verringerung der Insulindosierungen beim Patienten, so daß unerwünschte Nebenerscheinungen des Insulins vermieden werden können.

Die nachfolgenden Beispiele dienen zur weiteren Erläuterung der Erfindung anhand erfindungsgemäßer Stoffmischungen für therapeutische Behandlungszwecke.

Beispiel 1

Eine Infusionslösung für die Kalorienzufuhr wurde durch Auflösen von 250 g Glucose und 1 mg Prostaglandin $E_1$ in Wasser (aqua pro injectione) und Auffüllen der Literflasche bis zu einem Gesamtvolumen von 1000 ml hergestellt, worauf die Flasche sterilisiert wurde. Die Infusionslösung konnte mit einer Infusionsgeschwindigkeit von 500 ml in 3 Stunden appliziert werden, ohne daß eine unerwünschte Steigerung des Blutzuckerspiegels auftrat.

Beispiel 2

In gleicher Weise wurden Lösungen mit 10 mg Prostaglandin $E_2$ anstelle von Prostaglandin $E_1$ hergestellt.

Beispiel 3

Es wurden 100 g Glucose zusammen mit 2,2 g Isoleucin, 3,4 g Leucin, 3,7 g Lysin, 2,4 g Methionin, 2,3 g Phenylalanin, 1,9 g Threonin, 0,7 g Tryptophan, 2,0 g Valin, 4,3 g Arginin, 2,1 g Histidin, 1,1 g Asparaginsäure, 6,0 g Glutaminsäure, 3,8 g Glycin, 6,5 g Alanin, 0,4 g Tyrosin, 6,0 g Prolin, 2,0 g Serin, 1,5 g Asparagin, 0,2 g Cystin, 1,2 g Ornitin, 1,71 g KOH, 1,12 g Magnesiumacetat x $4H_2O$, 1,55 g NaOH, 1,99 g Aepfelsäure und 1 mg Prostaglandin $E_1$ in Wasser (aqua pro injectione) ad 1000 ml aufgelöst und wie üblich sterilisiert. Die Infusionslösung dient für die partielle parenterale Ernährung mit Aminosäurezufuhr zum Proteinaufbau und kann mit einer Infusionsgeschwindigkeit von 500 ml in 3 Stunden appliziert werden, ohne daß es zu einer unerwünschten Steigerung des Blutzuckerspiegels kommt.

0001124

**Beispiel 4**

400 mg Blutextrakt (Extr. sanguin, deprot. sicc.) und 1,5 mg Prostaglandin $E_1$ werden in 100 ml Celluloseglutcolat-Propylenglykol-Geleegrundlage eingerührt. Der erhaltene Gelee dient zur äußerlichen Anwendung bei offenen Hautdefekten.

**Beispiel 5**

100 mg Blutextrakt (Extr. sanguin. deprot. sicc.) und 0,5 mg Prostaglandin $E_1$ werden in 100 ml Polyäthylen-glykolcetylalkohol-Grundlage ad 100 ml eingerührt. Die erhaltene Creme dient zur äußerlichen Anwendung bei nicht nässenden Hautschäden.

**Beispiel 6**

0,5 g Blutextrakt (Extr. sanguin, deprot. sicc.) und 0,8 mg Prostaglandin $E_2$ werden in aqua pro injectione ad 250 ml gelöst. Die Lösung dient zur intravenösen Infusion bei einer Infusionszeit von ca. 30 Minuten.

**Beispiel 7**

100 mg Blutextrakt (Extr. sanguin, deprot. sicc.), 0,230 g NaCl und 100 ng Prostaglandin $E_1$ werden in aqua pro injectione ad 100 ml gelöst. Die erhaltene Lösung dient zur intraarteriellen Infusion.

0001124

Beispiel 8

Pulverisiertes N-(4-Methyl-benzol-sulfonyl-)N-n-butyl-harnstoff (Tolbutamid) und ein granuliertes Tablettenmaterial, das in 100 g 500 mg Prostaglandin $E_1$ enthält, wurden miteinander im Gewichtsverhältnis von 7:3 vermischt und diese Mischung zu 0,5 g schweren Tabletten verpreßt. Jede Tablette enthält somit 0,35 g Tolbutamid und 0,75 mg Prostaglandin $E_1$. Mit einer Dosierung von 1 bis 2 Tabletten morgens sowie am Abend konnte die diabetische Stoffwechsellage bei Patienten vom Erwachsenentyp gut eingestellt werden, wobei eine wesentlich geringere Menge an Sulfonylharnstoff erforderlich war als bei Applikation dieses oralen Antidiabetikums allein.

Beispiel 9

Zu 100 ml einer Insulinlösung (Altinsulin) mit 40 E/ml wurden 40 mg Prostaglandin $E_1$ hinzugefügt und die erhaltene Lösung in Ampullen zu je 10 ml abgefüllt.

EP-51 240

Patentansprüche

1. Arzneimittel zur Verbesserung der Glucoseverwertung in den Zellen und zur Verringerung des Blutzuckerspiegels, dadurch gekennzeichnet, daß es neben anderen Träger-substanzen und Wirkstoffen Prostaglandin $E_1$ und/oder $E_2$ enthält.

2. Arzneimittel nach Anspruch 1, dadurch gekennzeichnet, daß es als Infusionslösung Glucose, Fruktose, Xylit und/oder Aminosäuren sowie 0,3 bis 3 mg, vorzugsweise etwa 1 mg, Prosta-glandin $E_1$ oder 1,0 bis 20 mg, vorzugsweise etwa 10 mg, Prosta-glandin $E_2$ je Liter Infusionslösung enthält.

3. Arzneimittel nach Anspruch 2, dadurch gekennzeichnet, daß die Infusionslösung 100 bis 300 g Glucose, 50 bis 100 g Fruktose und/oder 50 bis 100 g Xylit je Liter Lösung enthält.

4. Arzneimittel nach Anspruch 1, dadurch gekennzeichnet, daß es enteiweißte Extraktstoffe des Blutes junger Kälber und die Prostaglandine $E_1$ und/oder $E_2$ enthält.

5. Arzneimittel nach Anspruch 4, dadurch gekennzeichnet, daß es für intravenöse Infusionen auf 250 ml Wasser 200 bis 1000 mg, vorzugsweise etwa 400 mg, Blutextraktstoffe sowie 0,45 bis 4,5 mg, vorzugsweise etwa 1,5 mg, Prostaglandin $E_1$ oder 0,1 bis

- 2 -

0001124

30 mg, vorzugsweise etwa 15 mg, Prostaglandin $E_2$ und für intra-arterielle Infusion auf 100 ml Wasser 50 bis 500 mg, vorzugsweise etwa 100 mg, Blutextraktstoffe sowie 40 bis 200 ng, vorzugsweise etwa 100 ng, Prostaglandin $E_1$ oder 0,05 bis 2,3 µg, vorzugsweise etwa 1 µg, Prostaglandin $E_2$ enthält.

6. Arzneimittel nach Anspruch 4, dadurch g e k e n n z e i c h - n e t , daß es als Gelee oder Salbe je 100 ml Basismaterial 50 bis 1000 mg Blutextraktstoffe sowie 0,1 bis 4,5 mg Prostaglandin $E_1$ oder 0,05 bis 30 mg Prostaglandin $E_2$ enthält.

7. Arzneimittel nach Anspruch 1, dadurch g e k e n n z e i c h - n e t , daß es ein orales Antidiabetikum, vorzugsweise ein Benzolsulfonylharnstoff-Derivat, insbesondere das Tolbutamid, sowie die Prostaglandine $E_1$ und/oder $E_2$ enthält, wobei das Prostaglandin $E_1$ in einer Menge von 30 mg bis 2,0 g und das Prostaglandin $E_2$ in einer Menge von 100 mg bis 10 g je 100 g Tolbutamid oder der äquivalenten Dosierung eines anderen oralen Antidiabetikums vorhanden ist.

8. Arzneimittel nach Anspruch 1, dadurch g e k e n n z e i c h - n e t , daß es Insulin sowie Prostaglandin $E_1$ und/oder $E_2$ enthält.

9. Arzneimittel nach Anspruch 8, dadurch g e k e n n z e i c h - n e t , daß es in 1 ml Injektionslösung ca. 40 E Insulin sowie 0,1 bis 1,2 mg Prostaglandin $E_1$ oder 1,0 bis 8 mg Prostaglandin $E_2$ enthält.

9121

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | <u>FR - A - 2 157 899</u> (UPJOHN)<br><br>* Seite 1, Zeile 1 - Seite 2, Zeile 32; Seite 8, Zeile 20 - Seite 11, Zeile 37; Seite 14, Zeile 28 - Seite 15, Zeile 5 *<br><br>--- | 1-3 |
| | <u>FR - A - 2 305 177</u> (UPJOHN)<br><br>* Seite 1, Zeile 1 - Seite 2, Zeile 38; Seite 9, Zeilen 23-33; Seite 14, Zeile 21 - Seite 16, Zeile 18 *<br><br>--- | 1-3 |
| | <u>US - A - 3 882 245</u> (DU CHARME)<br><br>* Spalte 3, Zeile 9 - Spalte 4, Zeile 14; Patentansprüche *<br><br>--- | 1-3 |
| | <u>AU - B - 419 560</u> (EARLE)<br><br>* Seite 1, Zeilen 1-24 *<br><br>--- | 1,8,9 |
| | <u>US - A - 3 851 052</u> (MONKHOUSE)<br><br>* Spalte 1, Zeilen 10-27 *<br><br>--- | 1,8,9 |
| | <u>US - A - 4 009 282</u> (VOORHEES)<br><br>* Patentansprüche *<br><br>--- | 1,6 |
| | CHEMICAL ABSTRACTS, Vol. 87, Nr. 13, 26. September 1977, Columbus Ohio, USA POLIS ET AL. "Normalization of the diabetic syndrom in hereditary<br><br>./. | 1 |

**KLASSIFIKATION DER ANMELDUNG (Int.Cl.²)**

A 61 K 31/20
31/64
37/02
37/26
9/00
9/06
9/20

**RECHERCHIERTE SACHGEBIETE (Int. Cl.²)**

A 61 K 31
37
9

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 28-11-1978 | JONAS |

EPA form 1503.1 06.78

Europäisches
Patentamt

EUROPÄISCHER RECHERCHENBERICHT

0001124

Nummer der Anmeldung
EP 78 10 0865
-2-

| | EINSCHLÄGIGE DOKUMENTE | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| | diabetic mice by PGBx, a polymeric derivative of prostaglandin B" & PHYSIOL.CHEM.PHYS. 1976 8(5), 429-436 <br><br> * Seite 148, Auszug Nr. 96 600r * <br><br> — | | |
| A | FR - A - 2 158 405 (UPJOHN) | 1 | |
| A | FR - A - 2 277 594 (PFRIMMER) | 2,3 | |
| A | FR - A - 2 274 279 (OTSUKA) | 2,3 | RECHERCHIERTE SACHGEBIETE (Int. Cl.³) |
| A | DE - A - 2 317 139 (KARL THOMAE) | 7 | |
| | — | | |